# EUROPÄISCHE PATENTSCHRIFT

(11) **EP 1 282 695 B1**
(45) Veröffentlichungstag und Bekanntmachung des Hinweises auf die Patenterteilung: **11.02.2009**
(21) Anmeldenummer: 00989856.0
(22) Anmeldetag: 14.11.2000
(51) Int. Cl.: C12N 11/08, G01N 33/545, A61L 33/00, A61L 33/12, A61L 29/08, A61L 29/16, A61L 31/08, A61L 31/16, A61L 27/28, A61L 27/54

(54) **BLUTKOMPATIBLE POLYMEROBERFLÄCHEN**
BLOOD-COMPATIBLE POLYMER SURFACES
SURFACES DE POLYMERE HEMOCOMPATIBLES

(30) Priorität: 17.11.1999 DE 19955341
(43) Veröffentlichungstag der Anmeldung: 12.02.2003
(73) Patentinhaber: JenAffin GmbH, 07745 Jena (DE)
(72) Erfinder: NOWAK, Götz, 99097 Erfurt (DE); BUCHA, Elke, 99094 Erfurt (DE)
(74) Vertreter: Vossius & Partner
(86) Internationale Anmeldenummer: PCT/EP2000/011253
(87) Internationale Veröffentlichungsnummer: WO 2001/036613

(56) Entgegenhaltungen:
- WO-A-95/07295
- WO-A-98/46648
- US-A- 5 053 453
- US-A- 5 342 693

## Beschreibung

Kunststoffe kommen in der Medizin auf vielfältige Art und Weise zum Einsatz. Die Untersuchung von Wechselwirkungen zwischen Kunststoffoberflächen und lebenden Zellen sowie die Verbesserung der Biokompatibilität solcher Oberflächen ist daher seit mehr als 30 Jahren Gegenstand intensivster Forschungstätigkeit. Trotzdem ist es bis heute noch nicht gelungen, Polymeroberflächen so zu gestalten, daß Blut bzw. Blutbestandteile nicht daran binden. Dies betrifft vor allem hochaktive Blutzellen, wie z.B. Thrombozyten, die nach der Anlagerung an die Kunststoffoberfläche zu Aktivierungsvorgängen, insbesondere zur Blutgerinnung, führen können.

Durch die Veränderung der Oberflächenladungen, durch Bildung von Mikrodomänenstrukturen, aber auch durch Einführung neuer Polymermischungen und Copolymere sind in diesem Bereich einige Fortschritte erzielt worden. Allerdings konnte ein Durchbruch zur Bereitstellung der benötigten dauerhaft blut- bzw. proteininerten Oberflächen noch nicht erreicht werden.

Um dieses Ziel zu erreichen, bedient sich die vorliegende Erfindung eines in der WO 98/46648 offenbarten Interaktionssystems, das die Anbindung von z.B. bioaktiven Substanzen an geeigneten Kunststoffoberflächen mittels spezieller Linker erlaubt. Dabei konnten durch die Verwendung von Inhibitoren der Blutplättchen- bzw. Zellaktivierung, die unter Einsatz dieser Linker auf Polymermaterialien immobilisiert wurden, wichtige Voraussetzungen für die Bereitstellung solcher blut- bzw. proteinkompatiblen Grenzschichten geschaffen werden. Im Rahmen der Erfindung konnte gezeigt werden, daß insbesondere Polysiloxane als Aktivierungshemmer für diese Anwendung zur Verfügung stehen. Durch das Aufbringen dieser Materialien auf Kunststoffoberflächen werden Blutbestandteile, insbesondere Proteine daran gehindert, ihrerseits Ablagerungen zu bilden. Von Silikonpolymeren ist bekannt, daß sie durch Aufbringen auf Glasoberflächen diesen eine erhöhte Blutkompatibilität verleihen. Versuche, die dabei eingesetzten Silikonöle in wirksamer Form auf Kunststoffoberflächen zu fixieren, schlugen jedoch bislang fehl.

Funktionelle Polymerobertlächen, die die Ausbildung der erfindungsgemäßen Grenzschichten erlauben, sind ebenfalls in WO 98/46648 beschrieben. Zum Einsatz kommen Homo- oder Copolymere, zu deren Herstellung mindestens ein Monomertyp eingesetzt wird, der neben einer polymerisierbaren Doppelbindung oder einer polykondensierbaren funktionellen Gruppe eine weitere Carbonylgruppe in Form eines Ketons oder eines Carbonsäurederivats enthält, die nicht an der Polymerisationsreaktion teilnimmt. Bevorzugt enthält das Polymer ein Strukturelement der Formel (A): 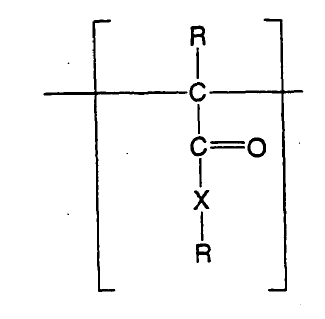 wobei die Reste R gleich oder verschieden sein können und einen Alkyl oder Arylrest oder ein Wasserstoffatom darstellen. Der Alkylrest kann linear oder verzweigt sein und besteht bevorzugt aus 1 bis 20 Kohlenstoffatomen. Der Arylrest besteht bevorzugt aus 6 bis 18, besonders bevorzugt aus 6 bis 12 Kohlenstoffatomen. Der Rest X ist fakultativ und bedeutet O, N oder CH₂. Für den Fall X = N trägt X zusätzlich zu dem in Formel (A) vermerkten einen weiteren Rest R, der unabhängig von den anderen Resten R wie vorstehend definiert ist.

Besonders bevorzugt als Alkylrest ist ein geradkettiger oder verzweigter, gegebenenfalls substituierter C₁₋₈-Alkylrest, beispielsweise ein Methyl-, Ethyl-oder Propylrest. Beispiele für gegebenenfalls vorhandene Substituenten umfassen ein oder mehrere Halogenatome, z.B. Fluor-, Chlor-, Brom- oder lodatome oder Hydroxylgruppen, C₁₋₆-Alkylreste oder C₁₋₆-Alkoxyreste oder C₁₋₆-Alkylthiolreste. Der Arylrest ist besonders bevorzugt ein monocyclischer oder bicyclischer, gegebenenfalls substituierter Arylrest, der gegebenenfalls ein oder mehrere Heteroatome enthalten kann. Beispiele für solche Arylreste sind Phenyl, 1- oder 2-Naphthyl, Indenyl- oder Isoindenylreste. Beispiele für heteroatomhaltige Arylreste sind C₃₋₉-Heteroarylreste, die Heteroatome, ausgewählt aus Sauerstoff-, Schwefel- oder Stickstoffatomen, enthalten. Monocyclische Heteroarylreste umfassen beispielsweise Pyrolyl-, Furyl-, Thienyl-, Imidazolyl-, N-Methylimidazolyl-, N-Ethylimidazolyl-, Benzothiazolyl-, Chinazolinyl-, Naphthylpyridinyl-, Chinolyinyl-, Isochinolinyl- und Tetrazolylreste.

Ein bevorzugtes Polymer, das im Rahmen der vorliegenden Erfindung eingesetzt werden kann, ist ein Polyalkylmethacrylat (PAMA) mit einem Alkylrest, der vorzugsweise 1-6 C-Atome umfaßt, wie z.B. Polymethylmethacrylat (PMMA), Polyethylmethacrylat (PEMA) oder Polypropylmethacrylat. Weiterhin können Polyvinylacetat, Polycyclohexylmethacrylate oder Polyphenylmethacrylat eingesetzt werden. Besonders bevorzugt wird jedoch Polymethylmethacrylat mit der erfindungsgemäßen blutinerten Grenzschicht versehen.

Auch Copolymere oder Polymermischungen aus beliebigen Anteilen der vorstehend genannten Polymere untereinander oder mit einer oder mehreren zusätzlichen Polymerkomponente(n), beispielsweise Polystyrol, Polyacrylnitril oder Polyamiden können eingesetzt werden. Bevorzugt beträgt der Anteil der Monomere oder Struktureinheiten, die eine Carbonylgruppe, bevorzugt ein Strukturelement (A) aufweisen an solchen Mischungen oder in solchen Copolymeren mindestens 20 %, besonders bevorzugt mindestens 40 % und ganz besonders bevorzugt mindestens 60 %.

Die Form der eingesetzten Oberfläche ist, wie in der WO 98/46648 beschrieben, in keiner Weise begrenzt, es können, z.B. flächige Strukturen, Hohlkörper sowie Mikropartikel und Kapillarstrukturen zum Einsatz kommen. Bevorzugt werden mikroporöse Kunststoffoberflächen verwendet, die die Bindung der Linker-Wirkstoff-Konjugate erleichtern. Selbstverständlich sollte beim medizinischen Einsatz auf die physiologische Verträglichkeit des eingesetzten Kunststoffs geachtet werden.

Eine Vielzahl von in der Medizin eingesetzten Gegenständen und Instrumenten wird aus den genannten Polymeren erzeugt und kann somit nach Aufbringen der hier offenbarten Grenzschicht in blutkompatibler Form bereitgestellt werden. Die Palette der in Frage kommenden Produkte umfasst dabei Kunststoffpartikel, Blutschlauchsysteme, Kathetermaterialien, Dialysatoren bzw. Dialysatormembranen aber auch Stent- und Implantationsmaterialien, die in der Ersatzchirurgie von Bedeutung sind. Neben Geräten, die innerhalb oder außerhalb des Körpers der Blutzirkulation ausgesetzt sind, können aber auch vorteilhaft Oberflächen beschichtet werden, die mit Blutproben in Kontakt kommen, oder die bei der Weiterbehandlung solcher Proben eingesetzt werden (z.B. Probengefäße, Rührgeräte). Produkte und Materialien können direkt vor ihrem Gebrauch oder bereits unmittelbar nach ihrer Herstellung mit einer blutinerten Grenzschicht überzogen werden.

Linker, die im Rahmen der vorliegenden Erfindung zur Immobilisation der Aktivierungs- bzw. Aggregationshemmer eingesetzt werden können, sind Moleküle, die mindestens zwei funktionelle Gruppen L1 und L2 aufweisen, die gleich oder unterschiedlich sein können. Eine dieser funktionellen Gruppen (L1) muss zur Bildung von Wasserstoffbrücken fähig sein und so die Bindung des Linkers an die Polymeroberfläche ermöglichen, was nicht ausschließt, daß sich auch andere Untereinheiten des Linkers, die eine geeignete elektronische oder räumliche Struktur aufweisen, an der Ausbildung dieser Bindung beteiligen. Die funktionelle Gruppe L2 wird so gewählt, dass eine Bindung zwischen dem Linker und der wirksamen Substanz hergestellt werden kann. Um unterschiedliche Wirksubstanzen gleichzeitig auf der Polymeroberfläche immobilisieren zu können, ist die gleichzeitige Verwendung mehrerer Linker mit unterschiedlichen Gruppen L2 möglich. Jedoch besteht auch die Möglichkeit, Linker eines Typs einzusetzen, die zwei oder mehr Gruppen L2 aufweisen, welche gleich oder unterschiedlich sind. Gleichermaßen können auch Linker eingesetzt werden, die mehrere gleiche oder unterschiedliche Gruppen L1 aufweisen. Bevorzugt sind L1 und L2 durch eine verzweigte oder geradkettige Alkylkette verbunden, die durch Heteroatome, bevorzugt Sauerstoffatome, unterbrochen ist.

Strukturelement L1 ist bevorzugt ein polares Wasserstoffatom, wie es beispielsweise in OH-, SH-, NH- oder PH-Bindungen vorliegt. Bevorzugt tragen die eingesetzten Linker als Strukturelement L1 eine Hydroxylgruppe. Das Strukturelement befindet sich bevorzugt an einer ausreichend wasserlöslichen Verbindung als Linker. Besonders bevorzugt ist L1 endständig am Linker angebracht.

Die funktionelle Gruppe, mittels derer eine Wirksubstanz, bevorzugt kovalent, an den Linker gebunden werden kann (L2) ist beispielsweise eine Hydroxyl- oder Carboxylgruppe, ein Succinimidylsuccinat, Succinimydylpropionat, Nitrophenylcarbonat, Trisylat, Epoxid, Aldehyd, Isocyanat oder ein Maleinimid. Weitere funktionelle Gruppen L2, mittels derer Linker zur Anbindung einer bioaktiven Substanz modifiziert bzw. aktiviert werden können, sind z.B. im Katalog der Firma Shearwater Polymers, Inc., 2307 Spring Branch Rd., Huntsville, AL 35801 (USA) beschrieben.

Anders als z.B. Enzyme als bioaktive Substanzen sind die zum Erreichen der Blutkompatibilität in der vorliegenden Erfindung eingesetzten Polyorganosiloxane vergleichsweise stabil. Sie können mit Linkern, die endständig mit einfachen funktionellen Gruppen versehen sind, wie z.B. Polyalkylenglykolen, ohne deren vorherige Aktivierung kovalent verknüpft werden. Bevorzugt besteht nach Verknüpfung des Linkers mit der bioaktiven Substanz zwischen beiden Komponenten eine Ether- oder eine Esterbindung.

Bevorzugt werden als Linker Polyalkylenglykole, Polyalkylenimine, Polyalkylenamine oder Polyalkylensulfide, sowie Polyoxazoline eingesetzt, wobei Polyalkylenglykole besonders bevorzugt sind. Der mittlere Polymerisationsgrad solcher Verbindungen liegt bevorzugt unterhalb von 50, besonders bevorzugt unterhalb von 30. Die Untergrenze liegt im allgemeinen bei einem Wert von 10, bevorzugt bei 20, wobei die bevorzugten Polymerisationsgrade innerhalb der vorgenannten Bereiche mit der Wahl der Linkergrundbausteine variieren können. Insbesondere bevorzugt werden Polyethylenglykole (PEG) eingesetzt, bei denen sowohl L1 als auch L2 Hydroxylgruppen sind. Die genannten Linker besitzen vorzugsweise ein Molekulargewicht von 1-50 kDa.

Als wirksame Verbindungen, welche die gewünschte Blutkompatibilität der Kunststoffoberfläche gewährleisten, werden im Rahmen der Erfindung Polyorganosiloxane eingesetzt, die linear oder verzweigt sein können. Als vorteilhaft hat sich die Verwendung von Poly(dialkylsiloxan) der Formel R₃SiO[R₂SiO]ₙSiR₃ erwiesen, wobei die Reste R gleich oder unterschiedlich sein können, und Wasserstoffatome oder Alkylreste mit 1 bis 8, bevorzugt 1 bis 4, besonders bevorzugt 1 bis 2 C-Atomen darstellen. n stellt eine natürliche Zahl dar, die so gewählt sein sollte, daß sich ein Viskositätsgrad des Siloxans ergibt, der zwischen 10 und 25000, bevorzugt zwischen 500 und 5000 mm²/s liegt. Aufgrund seiner bekannt guten physiologischen Verträglichkeit kommt insbesondere bevorzugt Dimethylpolysiloxan (Dimeticon) zum Einsatz. In diesem Fall ist R in der vorgenannten Formel CH₃, n liegt hier bevorzugt zwischen 1 und 50, besonders bevorzugt zwischen 1 und 20.

Die genannten Inhibitoren der Blutplättchen- bzw. Zellaktivierung werden mit den oben angeführten Linkern verbunden und anschließend als Linker-Wirkstoff-Konjugate mit der Kunststoffoberfläche in Kontakt gebracht. Auch hier bringt der Einsatz von Dimethylpolysiloxan Vorteile mit sich, da Polyethylenglykolverbindungen dieser Verbindung bereits kommerziell erhältlich sind, z.B. bei der Fa. Hüls unter den Produktnamen MN 4221, MN 4217, MN 4205 und MN 4211.

Nach der Verknüpfung des Aktivierungshemmers mit dem Linker wird das entstandene Konjugat mit der Polymeroberfläche verbunden. Die Bindung tritt bei bloßem Kontakt der Linker-Wirkstoff-Konjugate mit geeigneten Polymeroberflächen auf, ohne dass erhöhte Temperaturen oder der Einsatz von Katalysatoren oder anderer reaktionsbeschleunigender Reagentien nötig wäre. Sie kann z.B. durch Inkubation des Polymermaterials in einer bevorzugt wäßrigen Lösung der Konjugate erreicht werden. Die optimale Konzentration der Konjugate in der Lösung hängt z.B. von der Löslichkeit der verwendeten Komponenten und der zu erzielenden Oberflächenbelegung ab. Sie liegt jedoch häufig zwischen 0,1 µg/ml und 100mg/ml, bevorzugt zwischen 1 und 10 mg/ml. Nach einer Einwirkungszeit von wenigen Minuten und gegebenenfalls einer Spülung mit einer physiologischen Kochsalz- oder Pufferlösung ist die Silikonisierung der Oberfläche abgeschlossen.

Die entstehende Bindung der erzeugten Grenzfläche ist von ausgezeichneter Stabilität und kann in wässrigen Lösungen durch Verschiebungen des pH-Werts in einem Bereich von 2-13 nicht gelöst werden. Auch gegen das Spülen mit Salzlösungen hoher Ionenstärke (2n Glycin, 2n Harnstoff) ist die Bindung resistent. Sie kann damit unter physiologischen Bedingungen als irreversibel angesehen werden.

Die Bindungsdichte auf der beschichteten Oberfläche ist bereits nach ihrem Inkontaktbringen mit den Linker-Wirkstoff-Konjugaten unter Normalbedingungen, z.B. bei Raumtemperatur, bemerkenswert hoch. Eine derart vorbehandelter Kunststoff ist dazu in der Lage, die Aktivierung von zellulären Blutbestandteilen, speziell von Thrombozyten, aber auch die Bindung von Fibrinogen und anderen Proteinen an seine Oberfläche vollständig zu unterbinden. Weitere Untersuchungen haben jedoch ergeben, daß durch exogene Zufuhr von Energie, z.B. thermisch, wie durch Behandlung im Autoklaven (erhöhte Temperatur, Heißdampf), durch eine Erhöhung des Druckes oder durch Einwirkung von γ-Strahlen in einem Strahlensterilisationsgerät überraschenderweise die Belegungsdichte und die Festigkeit der Bindung noch einmal signifikant über das bei Normalbedingungen erhaltene Maß hinaus erhöht werden kann.

Weiter zeigte es sich, daß die erfindungsgemäßen Oberflächen auch bei nur teilweiser Belegung mit den Ligand-Wirkstoff-Konjugaten bereits die erforderliche blutneutrale Oberfläche aufweisen, ohne daß die Anbindung anderer mit Liganden versehener Wirksubstanzen nach dem aufgezeigten Prinzip verhindert wird. So ist bei einer Belegungsdichte von höchstens 50% der unter Normalbedingungen zu erzielenden Maximalbelegung eine hervorragende Blutkompatiblität der behandelten Oberfläche gewährleistet. Optimale Ergebnisse können sich, je nach Struktur des eingesetzten Siloxans, jedoch bereits bei deutlich niedrigeren Belegungsdichten von etwa 10% bis 20% zeigen. Durch Verwendung von Konjugatlösungen mit sehr geringen Konzentrationen kann z.B. die Belegungsdichte mittels Konzentrationslimitierung der Beschichtungsreaktion eingeschränkt werden. Dadurch steht eine für die in vivo-Anwendung im Vollblut wichtige biskompatible Oberfläche zur Verfügung, die dennoch zur in der WO 98/46648 offenbarten Präsentation und/oder Entfernung weiterer Linker-Wirkstoff-Konjugate zur Verfügung steht. So können z.B. pegylierte Wirkstoffe aus dem Blut entnommen werden, ohne daß es zu dessen Gerinnung an der exponierten Oberfläche kommt. Als zusätzliche bioaktive Wirk- oder Erkennungsstrukturen kommen z.B. Proteine, Nukleinsäuren, Oligo- oder Polynukleotide, Hormone, Enzyme, Antigene, Antikörper, Kohlenhydrate oder weitere zelluläre Signalstoffe und Immunbotenstoffe in Frage.

Die erfindungsgemäßen Grenzflächen können aber auch in der Implantationsmedizin zu erheblichen Verbesserungen bei der Verträglichkeit der eingesetzten Materialien beitragen, da damit auch an den Gewebs- und Blutgrenzflächen schädliche Interaktionen, z.B. unspezifische Entzündungen, unterbunden werden können. Die gleichzeitige Möglichkeit, hier trotzdem gezielt mit Linkern verknüpfte Wirkstoffe dem Biomikroenvironment zu präsentieren, ergibt vollkommen neue Ansatzpunkte für eine langfristige Anwendung solcher Materialien.

Die den erfindungsgemäßen Oberflächen eigene Blut- bzw. Proteinresistenz über lange Anwendungszeiträume ist jedoch nicht nur für medizinische Werkstoffe, sondern auch für medizinische Geräte und Werkzeuge von Bedeutung. So erlaubt es die Erfindung, die Proteinisierung solcher Geräte, z.B. von Langzeitkathetern, zu verhindern, die einen idealen Nährboden für Bakterien liefert und so sekundären Infektionen Vorschub leistet. Damit bietet die hier erfindungsgemäße Grenzschicht die Möglichkeit, geeignete Kunststoffmaterialen in antibakteriell bzw. antimikrobiell modifizierter Form einzusetzen.

Neben ihrer vollständigen Kompatibilität mit proteinhaltigen Proben, insbesondere Blutproben oder zirkulierendem Vollblut sind die modifizierten Kunststoffoberflächen der vorliegende Erfindung für die Präsentation von immobilisierten Wirkstoffen, aber auch für die Anbindung und/oder Entfernung von linker-gekoppelten Wirksubstanzen oder Erkennungsstrukturen geeignet. Wirkstoffe können so unmittelbar in die Blutbahn eingebracht oder daraus, nach Kopplung mit einem entsprechenden Linker, entfernt werden. Die erfindungsgemäßen Oberflächen eröffnen damit sowohl in der Therapeutik wie auch in der Diagnostik, aber auch in verwandten Gebieten, wie der Diäthetik, vielfältige neue Indikationen und Anwendungsbereiche.

Die folgenden Beispiele illustrieren die Wirksamkeit der erfindungsgemäßen Oberflächenbeschichtungen.

Zur Anwendung kommen dabei Polymethylmethacrylatpartikel, monodispers, in der Größe von 5,9 bis 6,1 µm (Microparticles GmbH) Berlin) sowie kommerzielle Dialysatoren der Serie BK 05 der Fa. Toray Industries, Tokio, (OF 0,5 m²) bzw. hieraus gefertigte experimentelle Mikrodialysatoren mit einer Oberfläche von 100 cm².

### Versuchsbeispiel 1:

50 µl einer 5%igen Polymethylmethacrylatpartikellösung (Partikeldurchmesser 5,9-6,1 µm) werden mit PEG-Dimethylpolysiloxan (MN 4205) in einer Konzentration von 1 µg/ml für 10 min in einem Rollschüttler vermischt. Anschließend werden durch Kurzzentrifugation bei 1000 g für 3 min die Partikel sedimentiert und die überstehende Lösung entfernt. Danach werden die Partikel mit jeweils 1 ml Tyrodelösung aufgenommen, für kurze Zeit geschüttelt und nach Sedimentierung noch einmal mit Tyrode gewaschen und für die weitere Verwendung in Tyrodelösung aufbewahrt. R-Hirudin in einer Konzentration von 300 µg/ml wird mit 10 ml Vollblut in einem Reagenzglas vermischt. Das frisch abgenommene Humanblut wird mit den sedimentierten Partikeln, welche vorher mit PEG-Dimethylpolysiloxan versetzt wurden, vermischt. Nach 15, 20, 30 und 40 min werden die in einem Rollmischer gut bewegten Proben zur Plättchenmessung in einen CellDyn 2000-Analysator appliziert und die Plättchenzahl ermittelt. Zum Vergleich werden Partikel benutzt, die nicht mit PEG-Dimethylpolysiloxan beschichtet wurden. Es konnte gezeigt werden, daß in der Suspension mit nicht beschichteten Partikeln eine große Anzahl der zirkulierenden Blutplättchen gebunden wird. Es kommt innerhalb der ersten 10 min in der Probe zu einem steilen Abfall der Plättchenzahl, die nur geringfügig durch gegenläufige Desaggregationsvorgänge nach diesem Zellzahltief wieder steigt. Trotzdem bleibt die größte Anzahl der Plättchen in der Blutprobe an der Polymeroberfläche haften (>90%). Im Gegensatz dazu sind in der Suspension mit den Partikeln, die mit Dimethylpolysiloxan beschichtet wurden, diese Plättchenzahlveränderungen nicht nachweisbar. Während der gesamten Versuchsdauer sind die Plättchenzahlen in Höhe der Ausgangswerte nachzuweisen.

### Versuchsbeispiel 2:

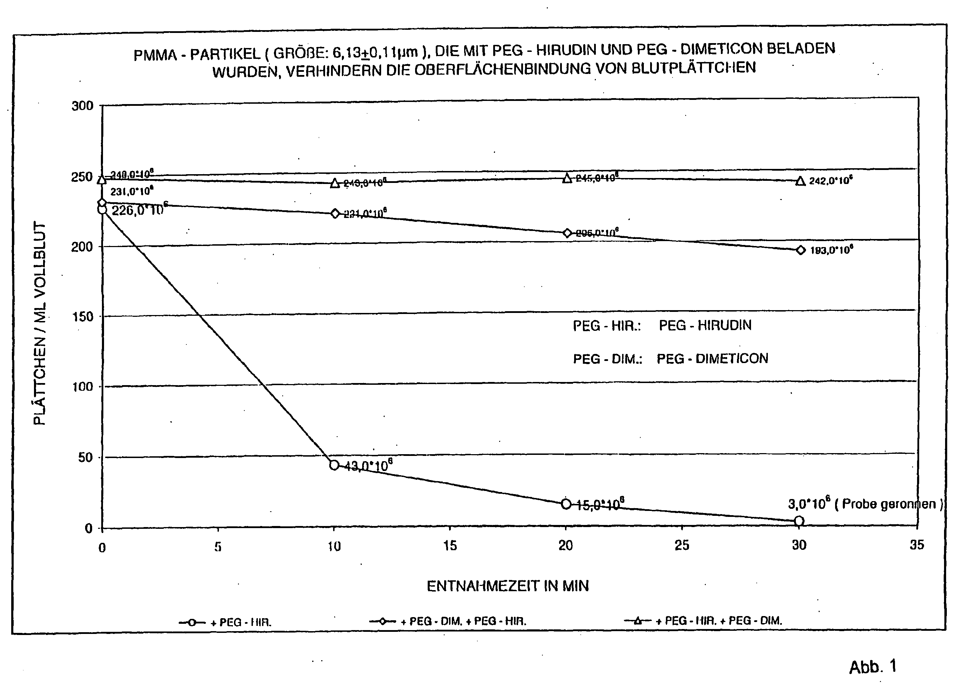

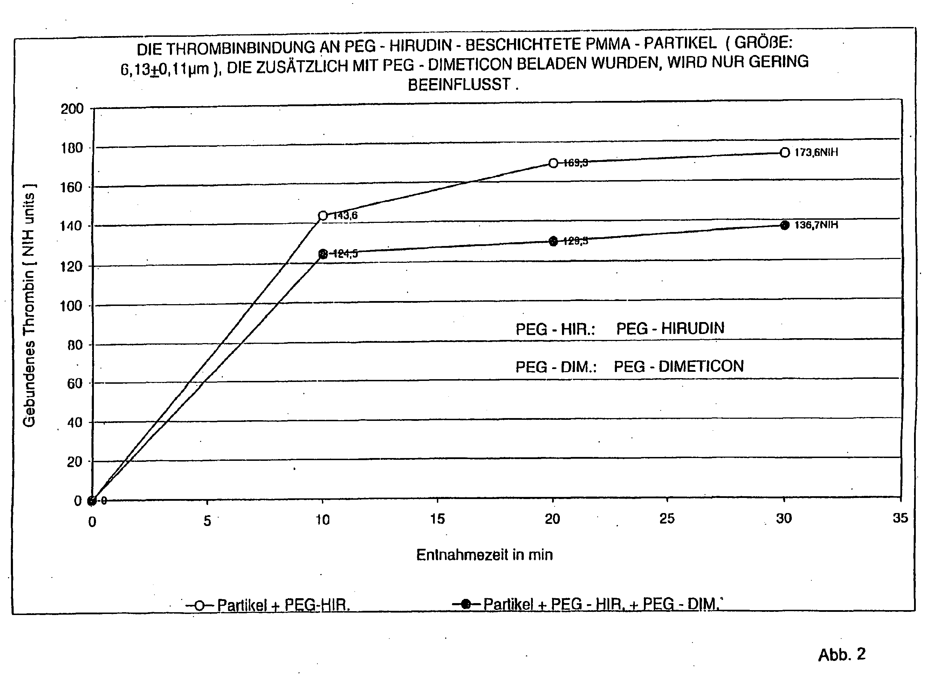

Eine weitere Versuchsreihe wurde mit Versuchsbedingungen durchgeführt, die mit denen des Beispiel 1 identisch sind, mit der Ausnahme, daß das Blut mit PEG-Hirüdin antikoaguliert wurde. In Abb. 1 sind die entsprechenden Ergebnisse dargestellt. Die Blutplättchen werden in der Partikelsuspension ohne PEG-Dimethylpolysiloxan-Beschichtung innerhalb von 10-15 min nach Zugabe zum PEG-Hirudin-antikoagulierten Blut nahezu vollständig an den Partikeln adhäriert. Innerhalb von 30 min kommt es zum Gerinnen der Probe, da das PEG-Hirudin komplett an den Partikeln gebunden wurde. Bei Vorbehandlung der Partikel mit PEG-Dimethylpolysiloxan ist der Plättchenabfall total aufzuhalten, aber auch in diesem Versuch ist die gut gemischte Probe nach etwa 40 min geronnen. Bei Messung der Hirudinkonzentration in den Proben ist erkennbar, daß im Gegensatz zu den r-Hirudin-behandelten Proben, in denen während des gesamten Versuchszeitraumes über 60 min eine konstante Hirudinblutkonzentration von 25-30 µg/ml r-Hirudin nachweisbar ist, in den PEG-Hirudin-Proben eine kontinuierliche Abnahme der PEG-Hirudin-Konzentration nachweisbar ist. Nach 30 min ist der PEG-Hirudingehalt auf 3 µg/ml abgefallen, nach 40 min ist in den Proben kein PEG-Hirudin mehr enthalten. Aus den hier vorgestellten Untersuchungen ist klar ersichtlich, daß durch die Vorbehandlung der Mikropartikel mit PEG-Dimethylpolysiloxan in einem Konzentrationsbereich zwischen 0,1 und 1 µg/ml die Adsorption von Plättchen verhindert wird. Trotzdem ist die Bindung von pegyliertem Hirudin an der Partikeloberfläche nicht gestört. Beim Vergleich mit in vitro-Untersuchungen; bei denen PEG-Hirudin zu den Mikropartikellösungen hinzugegeben wird, ist eine Verminderung der PEG-Hirudinbindungskapazität durch die PEG-Dimethylpolysiloxan-Vorbehandlung nicht nachweisbar. Weiterhin wurde untersucht, ob die Thrombinbindung an den Partikeln, die nach der PEG-Dimethylpolysiloxan-Vorbehandlung zusätzlich PEG-Hirudin gebunden haben, noch erhalten geblieben ist. Ein Versuchsbeispiel hierzu ist in Abb. 2 dargestellt. Es kann zweifelsfrei gezeigt werden, daß die Thrombinaffinität nahezu vollständig erhalten blieb.

### Versuchsbeispiel 3:

Experimentelle PMMA-Dialysatoren mit einer Oberfläche von 100 cm² werden mit Dimethylpolysiloxanlösungen in einer Konzentration von 1 µg/ml mit Hilfe einer in vitro-Zirkulationseinrichtung für 10 min gespült und danach mit Tyrodelösung gewaschen. Anschließend werden die mikrokapillären Dialysatoren mit PEG-Hirudin-antikoaguliertem Vollblut (50 µg/ml) in einem Rezirkulationsmodell behandelt. Es kann auch in diesem Versuchsansatz zweifelsfrei gezeigt werden, daß trotz Anbindung von PEG-Hirudin an die kapillären Oberflächen eine Verminderung der Plättchenzahlen nicht zustandekommt. Obwohl das PEG-Hirudin zum größten Teil auf der PMMA-Oberfläche der Dialysatoren gebunden ist, sind die Systeme über mehr als 30 min ohne wesentlichen Druckanstieg rezirkulierbar.

## Patentansprüche

1. Blutkompatible Oberfläche, umfassend eine Polymeroberfläche, die gleiche oder unterschiedliche Struktureinheiten enthält, die-Carbonylgruppen tragen, sowie eine Vielzahl darauf immobilisierter Linker-Wirkstoff-Konjugate, wobei die Linker ein Strukturelement enthalten, das zur Ausbildung einer Wasserstoffbrückenbindung fähig ist und so die Bindung an die Polymeroberfläche ermöglicht, und gleich oder unerschiedlich ausgewählt sind aus Polyalkylenglykol, Polyalkylenimin, Polyalkylenamin, Polyalkylensulfid und Polyoxazolin und wobei als Wirkstoff ein Polyorganosiloxan mit den Linkern verknüpft ist.

2. Oberfläche gemäß Anspruch 1, wobei die Polymeroberfläche eine Vielzahl von Struktureinheiten aufweist, die gleich oder unterschiedlich sind, ausgewählt aus Polyalkylmethacrylat-, Polyvinylacetat-, Polycyclohexylmethacrylat- und/oder Polyphenylmethacrylateinheiten.

3. Oberfläche gemäß einem der Ansprüche 1 oder 2, wobei als Wirkstoff Dimethylpolysiloxan eingesetzt wird.

4. Oberfläche gemäß einem der Ansprüche 1 bis 3, umfassend freie Koordinationsstellen zur Immobilisation weiterer Linker-Wirkstoff-Konjugate.

5. Oberfläche gemäß einem der Ansprüche 1 bis 4, umfassend zusätzliche Linker-Wirkstoff-Konjugat, deren Wirkstoffe der Oberfläche neben ihrer Blutkompatibilität weitere Bioaktivität verleihen.

6. Kunststoffgegenstand, umfassend eine blutkompatible Oberfläche gemäß einem der Ansprüche 1 bis 5.

7. Medizinisches Gerät oder Werkzeug, umfassend eine blutkompatible Oberfläche gemäß einem der Ansprüche 1 bis 5.

8. Gerät oder Werkzeug nach Anspruch 7 in Form eines Probengefäßes, eines Blutschlauchs, eines Katheters, eines Dialysators oder Bestandteilen davon.

9. Stent- oder Implantationsmaterial, umfassend eine blutkompatible Oberfläche gemäß einem der Ansprüche 1 bis 5.

10. Verfahren zur Herstellung einer blutkompatiblen Oberfläche nach einem der Ansprüche 1 bis 5, umfassend das Inkontaktbringen der Polymeroberfläche mit den Linker-Wirkstoff-Konjugaten.

11. Verfahren gemäß Anspruch 10, wobei die Polymeroberfläche in einer lösung der Linker-Wirkstoff-Konjugate inkubiert wird.

12. Verfahren gemäß Anspruch 10 oder 11, wobei die Konzentration der Linker-Wirkstoff-Konjugate so begrenzt wird, daß die entstehende blutkompatible Oberfläche freie Koordinationsstellen aufweist.

13. Verfahren gemäß Anspruch 10 oder 11, zusätzlich umfassend die Zufuhr exogener Energie während oder nach der Belegung der Oberfläche, um so die Belegungsdichte zu erhöhen.

14. Verfahren gemäß Anspruch 13, wobei die Energiezufuhr durch hohe Drücke thermisch oder mittels γ-Strahlung verfolgt.

15. Diagnoseverfahren, umfassend das Inkontaktbringen einer Blutprobe mit einer Oberfläche gemäß einem der Ansprüche 1 bis 5.

16. Verwendung eines Materials mit einer blutkompatiblen Oberfläche gemäß einem der Ansprüche 1 bis 5 bei der Lagerung, beim Transport, oder der Untersuchung von Humanblut außerhalb des menschlichen Körpers.

17. Verwendung nach Anspruch 16, zusätzlich umfassend die Zugabe oder Entfernung von Blutbestandteilen oder Wirkstoffen.

## Claims

1. Blood-compatible surface comprising a polymer surface containing the same or different structural units that carry carbonyl groups, and a plurality of conjugates of linkers and active agents immobilised thereon, wherein said linkers contain a structural element that is able to form a hydrogen bond, thus enabling a bonding to the polymer surface, and the linkers are the same or different and are selected from the group consisting of polyalkylene glycol, polyalkylene imine, polyalkylene amine, polyalkylene sulfide and polyoxazoline, and wherein a polyorganosiloxane acting as the active agent is linked to the linkers.

2. The surface as claimed in claim 1, wherein said polymer surface contains a plurality of structural units which are the same or different and are selected from the group consisting of polyalkyl methacrylate, polyvinyl acetate, polycyclohexyl methacrylate and/or polyphenyl methacrylate units.

3. The surface as claimed in any one of claims 1 or 2, wherein dimethyl polysiloxane is used as the active agent.

4. The surface as claimed in any one of claims 1 to 3, comprising free coordination sites for the immobilisation of further conjugates of linkers and active agents.

5. The surface as claimed in any one of claims 1 to 4, comprising additional conjugates of linkers and active agents, said active agents providing the surface with further bioactivity in addition to blood compatibility.

6. Plastic article comprising a blood-compatible surface as claimed in any one of claims 1 to 5.

7. Medical device or instrument comprising a blood-compatible surface as claimed in any one of claims 1 to 5.

8. The device or instrument as claimed in claim 7 in the form of a sample container, a blood tube, a catheter, a dialyser or components thereof.

9. Stent or implantation material comprising a blood-compatible surface as claimed in any one of claims 1 to 5.

10. Process for the preparation of a blood-compatible surface as claimed in any one of claims 1 to 5 comprising contacting the polymer surface with said conjugates of linkers and active agents.

11. The process as claimed in claim 10, wherein said polymer surface is incubated in a solution of said conjugates of linkers and active agents.

12. The process as claimed in claim 10 or 11, wherein the concentration of said conjugates of linkers and active agents is limited in such a way that the resulting blood-compatible surface exhibits free coordination sites.

13. The process as claimed in claim 10 or 11, additionally comprising the supply of exogenous energy during or after coverage of the surface so as to increase the coverage density.

14. The process as claimed in claim 13, wherein energy is supplied by high pressures, thermally or via γ-rays.

15. Diagnostic method comprising contacting a blood sample with a surface as claimed in any one of claims 1 to 5.

16. Use of a material having a blood-compatible surface as claimed in any one of claims 1 to 5 in the storage, in the transport or in the testing of human blood outside the human body.

17. Use according to claim 16, additionally comprising the addition or removal of blood constituents or active agents.

## Revendications

1. Surface compatible avec le sang, comprenant une surface polymère qui contient des unités structurelles identiques ou différentes, qui portent des groupements carbonyle, ainsi qu'une pluralité de conjugués de type lieur-principe actif immobilisés sur celle-ci, dans laquelle les lieurs contiennent un élément de structure, qui est en mesure de former une liaison hydrogène pontée et qui permet, par suite, la liaison à la surface polymère, et sont choisis de manière identique ou différente parmi le polyalkylèneglycol, la polyalkylène-imine, la polyalkylénamine, le poly(sulfure d'alkylène) et la polyoxazoline, et dans laquelle un polyorganosiloxane est relié aux lieurs, comme principe actif.

2. Surface selon la revendication 1, dans laquelle la surface polymère présente une pluralité d'unités structurelles, qui sont identiques ou différentes et qui sont choisies parmi les unités poly-(méthacrylate d'alkyle), poly(acétate de vinyle), poly(méthacrylate de cyclohexyle) et/ou poly-(méthacrylate de phényle).

3. Surface selon l'une quelconque des revendications 1 ou 2, dans laquelle on utilise le diméthylpolysiloxane comme principe actif.

4. Surface selon l'une quelconque des revendications 1 à 3, comprenant des sites de coordination libres en vue d'immobilisation d'autres conjugués de type lieur-principe actif.

5. Surface selon l'une quelconque des revendications 1 à 4, comprenant d'autres conjugués de type lieur-principe actif, dont les principes actifs confèrent à la surface une autre bioactivité, en plus de leur compatibilité avec le sang.

6. Objet en matériau synthétique, comprenant une surface compatible avec le sang selon l'une quelconque des revendications 1 à 5.

7. Appareil ou outil médical, comprenant une surface compatible avec le sang selon l'une quelconque des revendications 1 à 5.

8. Appareil ou outil selon la revendication 7, se présentant sous la forme d'un récipient à échantillon, d'un tuyau pour le sang, d'un cathéter, d'un dialyseur ou de ses composants.

9. Matériau d'endoprothèse ou d'implantation, comprenant une surface compatible avec le sang, selon l'une quelconque des revendications 1 à 5.

10. Procédé de fabrication d'une surface compatible avec le sang selon l'une quelconque des revendications 1 à 5, comprenant la mise en contact de la surface polymère avec les conjugués de type lieur-principe actif.

11. Procédé selon la revendication 10, dans lequel la surface polymère est incubée dans une solution contenant les conjugués de type lieur-principe actif.

12. Procédé selon la revendication 10 ou 11, dans lequel la concentration des conjugués de type lieur-principe actif est limitée, de sorte que la surface résultante compatible avec le sang présente des sites de coordination libres.

13. Procédé selon la revendication 10 ou 11, comprenant, en outre, l'alimentation d'énergie exogène pendant ou après l'étape de charge de la surface, dans le but d'augmenter la densité de charge.

14. Procédé selon la revendication 13, dans lequel l'alimentation d'énergie est effectuée sous des pressions élevées par voie thermique ou au moyen d'un rayonnement de type γ.

15. Procédé de diagnostic, comprenant la mise en contact d'un échantillon sanguin avec une surface selon l'une quelconque des revendications 1 à 5.

16. Utilisation d'un matériau ayant une surface compatible avec le sang selon l'une quelconque des revendications 1 à 5, dans le cadre de la conservation, du transport ou de l'analyse de sang humain extrait du corps humain.

17. Utilisation selon la revendication 16, comprenant également l'addition ou l'élimination de composants sanguins ou de principes actifs.
